# EUROPÄISCHE PATENTSCHRIFT

(11) **EP 0 948 316 B1**
(45) Veröffentlichungstag und Bekanntmachung des Hinweises auf die Patenterteilung: **06.08.2003**
(21) Anmeldenummer: 97925943.9
(22) Anmeldetag: 26.05.1997
(51) Int. Cl.: A61K 9/00, A61K 9/12

(54) **MITTEL ZUR ERZEUGUNG EINES LANGANHALTENDEN SÄTTIGUNGSEFFEKTS**
AGENT FOR PRODUCING A LONG-LASTING SATURATION EFFECT
AGENT POUR PRODUIRE UN EFFET DE SATURATION DE LONGUE DUREE

(30) Priorität: 27.11.1996 WO PCT/EP96/05240
(43) Veröffentlichungstag der Anmeldung: 13.10.1999
(73) Patentinhaber: Beisel, Günther, 40789 Monheim (DE)
(72) Erfinder: Beisel, Günther, 40789 Monheim (DE)
(74) Vertreter: Fitzner, Uwe, Dr.
(86) Internationale Anmeldenummer: EP9702676
(87) Internationale Veröffentlichungsnummer: WO98023259

(56) Entgegenhaltungen:
- EP-A- 0 449 592
- EP-A- 0 568 334
- WO-A-97/20562
- DE-C- 4 119 140
- US-A- 4 298 002
- Dialog Information Services, file 351, DERWENT WPI, Dialog accession no. 002537890, WPI accession no. 80-55915, LION DENTIFRICE CO LTD: "Sponge-like medicinal base - comprising mixt. of polymers soluble in water or converted to viscous gels and water soluble non-volatile plasticiser"; & JP,A,55083715, 19800624
- Chemical Abstracts, Band 96, Nr. 26, 28 Juni 1982, (Columbus, Ohio, US), Seite 378, Zusammenfassung 223289j; & JP,A,8206403, (Lion Corp.) 4 Februar 1982
- Dialog Information Services, file 351, DERWENT WPI, Dialog accession no. 003193628, WPI accession no. 81-54181, LION DENTIFRICE CO LTD: "Preparation for admin. to coelom - comprising polymer sponge contg. oil-soluble adhesive polymer"; & JP,A,56068608, 19810609

## Beschreibung

Die vorliegende Erfindung betrifft ein Mittel zur oralen Einnahme, enthaltend ein in Wasser und gastrointestinalen Flüssigkeiten unlösliches oder schwer lösliches Material in Form eines schwammartigen Gebildes, ein Verfahren zu dessen Herstellung und dessen Verwendung zur Herstellung von Mitteln zur Erzeugung eines Sättigungseffekts sowie zur Herstellung von oral verabreichbaren Arzneimitteln, Nahrungsergänzungsmittlen oder Nahrungsmitteln mit zeitverzögerter und schonender Wirkstofffreisetzung.

Es sind zahlreiche Versuche unternommen wurde, auf medikamentösem Weg überflüssige Fettanreicherungen im menschlichen Körper abzubauen beziehungsweise deren Entstehung zu verhindern. Es gibt z.B. sogenannte Appetitzügler, die den Körper auf biochemischem Weg eine Abneigung zur Nahrungsaufnahme zu suggerieren versuchen. Diese Mittel haben zum Teil erhebliche schädliche Nebenwirkungen.

Neben den zahlreichen bekannten Diätvorschlägen gibt es auch mechanische und elektromechanische Mittel, mit denen ein gezielter Fettabbau beziehungsweise Muskelaufbau erfolgen soll. Die Wirkung solcher Mittel ist jedoch sehr zweifelhaft.

Aus der deutschen Patentschrift DE 4025912 ist ein Mittel zur oralen Einnahme bekannt, das aus einem im Magen lösbaren und den Inhalt freigegebenden Behälter besteht. Dieser ist mit einem Stoff gefüllt, der nach seinem Freisetzen im Magen sein Volumen vergrößert und dadurch dem Körper ein Sättigungsgefühl suggeriert.

Nachteilig bei diesem System ist, daß die Herstellung einer speziellen Umhüllung erforderlich ist. Bevor das Mittel seine Wirkung im Magen entfalten kann, vergeht demgemäß einige Zeit. Zudem ist die Herstellung der Umhüllung und der komprimierten Form des Mittels mit erheblichem Aufwand verbunden.

Aufgabe der vorliegenden Erfindung ist es demgemäß, ein Mittel mit Sättigungswirkung zur oralen Einnahme zur Verfügung zu stellen, das die geschilderten Nachteile der bisherigen Mittel nicht aufweist. Insbesondere soll der Aufwand für die Herstellung der komprimierten Form verringert und ein Lebensmittelähnlicher Verzehr ermöglicht werden.

Diese Aufgabe wird dadurch gelöst, daß das Mittel aus einem elastischen Material besteht,
- das aus einem elastischen Material besteht,
- das durch Kau- und Gaumenschluckbewegung verformbar, formreduzierbar und komprimierbar ist, so daß es die Speiseröhre passieren kann
- das nach dem Verlassen der Speiseröhre in Trinkflüssigkeiten und in gastrointestinalen Flüssigkeiten im Magen, Volumen aufbauend dekomprimierbar ist,
- das während des Magenaufenthaltes einen physiologischen Sättigungseffekt erzeugt, und verzögert eingebundene wirk- und Inhaltsstoffe freigibt, und
- das nach mehrstündigem Aufenthalt im Magen unresorbiert oder gering resorbierbar über den Darm den Körper wieder verläßt.
Mit anderen Worten, das Material wird mit einer einfachen Kau- und Schluckbewegung und Unterstützung durch Flüssigkeitseinnahme bei Durchtritt durch die menschliche Speiseröhre komprimiert und nach dem Verlassen der Speiseröhre in Wasser sowie gastrointestinalen Flüssigkeiten im Magen dekomprimiert, wo es sein ursprüngliches Raumvolumen weitgehend wiedererhält.

Unter schwammartigen Gebilden sind erfindungsgemäß Schäume zu verstehen, die aus gasgefüllten, kugel-polyederförmigen Zellen bestehen, welche durch hochviskose oder feste Zellstege begrenzt sind. Einsetzbar sind erfindungsgemäß sowohl natürlich vorkommende Schwämme als auch synthetisch hergestellte schwammartige Gebilde.

Die Herstellung der schwammartigen bzw.-förmigen Gebilde erfolgt mit an sich bekannten Methoden nach dem Stand der Technik. In Abhängigkeit von dem eingesetzten Ausgangsmaterial kann im einfachsten Falle ein Schaum durch Einblasen, durch Schlagen, Schütteln, Verspritzen oder Rühren in der betreffenden Gasatmosphäre erhalten werden. Bei den Polymeren entsteht die Schaumstruktur aufgrund chemischer Reaktionen. So werden bei den Polyurethanen durch Zugabe von Blähmitteln, die sich bei bestimmter Temperatur während der Verarbeitung unter Gasbildung zersetzen, oder durch Zusatz von flüssigen Lösemitteln während der Polymerisation geschäumt. Die Verschäumung erfolgt entweder beim Verlassen des Extrusionswerkzeuges, d.h. im Anschluß an das Extrudieren oder Spritzgießen oder in offenen Formen. Die Härtung erfolgt unter den für die jeweilige chemische Verbindung des Materials charakteristischen Bedingungen.

Unabdingbare Voraussetzung für die Einsetzbarkeit des erfindungsgemäßen Materials und der Schwammstruktur ist, daß das Material komprimierbar ist, ohne daß die Zellstege brechen. Um das erfindungsgemäße Material nämlich für die oralen Einnahme einsetzen zu können, muß das schaumförmige bzw.artige Material bei Durchtritt durch die Speiseröhre sich ohne weiteres komprimieren lassen. Insbesondere darf es beim Passieren der Speiseröhre nicht zu Beschwerden kommen.

Für die Auswahl des Materials und die Art der Schaumbildung ist ferner wesentlich, daß es quellfähig bleibt, ohne daß die Zellstege zerstört werden. Nach dem Durchtritt durch die Speiseröhre soll das schwammartigen Gebilde wenigstens wieder die Größe annehmen, die es vor dem Eintritt in die Speiseröhre hatte. Gegebenenfalls kann das Material auch zu einer Größe quellen, die über die ursprünglichen Volumina hinausgeht.

Das Volumen des dekomprimierten, schwammartigen Gebildes liegt bei 2, vorzugsweise 40 cm³. Das Volumen im komprimierten Zustand liegt bei 0,5, vorzugsweise 3 cm³.

Das schwammartige Gebilde kann im dekomprimierten Zustand jede beliebige Form haben. Bevorzugt sind jedoch quaderförmige oder rechtecksförmige oder runde Ausgestaltungen. Die Oberflächen liegen in diesem Fall bei 6 bis 60 cm².

Vorzugsweise ist das Material so ausgelegt, daß das schwammartige Gebilde auf 1/2 bis 1/20 vorzugsweise 1/4 bis 1/10 seines Volumens bzw. seiner Größe komprimierbar ist. Unter physiologischen Bedingungen soll das durch Kauund Schluckbewegung formreduzierte und Komprimierte Material sich nach Austritt aus der Speiseröhre vorzugsweise auf das Zwei- bis zwanzigfache, besonders bevorzugt auf das Vier-bis zehnfache seines Volumens im Magen ausdehnen können.

Als Material für das schwammartige Gebilde können erfindungsgemäß natürliche, halbsynthetische oder synthetische Polymere zum Einsatz kommen. Beispiele geeigneter synthetischer Polymere sind Polyurethane, Polyacrylate, Poly(meth)acrylsäureester, Homo- und Copolymere des Vinylacetats. Zu den natürlichen und halbsynthetischen Polymeren zählen u.a. Cellulose, Ether, Diethylcellulose oder Celluloseester, wie Cellulosediacetat, Cellulosetriacetat, Celluloseacetat-Propionat und Celluloseacetat-Butyrat. Erfindungsgemäß geeignet sind z.B. Cellulosederivate, insbesondere entsprechende Ether, z.B. Methylcellulose, Hydroxypropylcellulose, Hydroxypropyl-methylcellulose, oder Natriumcarboxymethylcellulose (vorzugsweise solche Verbindungen mit höherer Viskosität); gewisse Polymere, wie Polyacrylsäure und Salze davon; natürliche (anionische) Schleimstoffe, z.B. Xanthan Gummi, Guar Gummi, Traganth oder Alginsäure und Salze davon, und dergleichen Darüber hinaus ist auch der Einsatz unlöslicher Polysacharide, wie Chitin bzw. Chitinderivate oder mikrokristalliner Cellulose denkbar. Erfindungsgemäß besonders bevorzugt werden linerare hochmolekulare Polymere. Vor allem sind erfindungsgemäß solche Polymere einsetzbar, die Faserstruktur besitzen. Beispiele für solche Stoffe sind die Skleroproteine, wie Keratine, Conchagene, Fibrin, Elastine, Chitin und Collagen. Letzteres wird erfindungsgemäß besonders bevorzugt.
Das erfindungsgemäße Mittel kann u.a. auch pharmazeutisch wirksame Stoffe, Nahrungsmittel bzw. Nahrungsergänzungsmittel-Mineralstoffe, z.B. Vitamine, Ballaststoffe, Eiweiße sowie andere Lebensmittelstoffe oder Aromastoffen enthalten.

Neben den genannten Stoffen können dem Trägermaterial auch weitere Hilfsstoffe beigefügt werden. Unter anderem können im Falle des Einsatzes von pharmazeutisch wirksamen Substanzen noch zusätzlich retardierende Stoffe in Frage kommen.

Außerdem können die Mittel gemäß der vorliegenden Erfindung zusätzlich Füll-, Spreng-, Binde- und Gleitmittel sowie Trägerstoffe enthalten.

Die vorliegende Erfindung betrifft auch ein Verfahren zur Herstellung des oben beschriebenen Mittels. Dabei wird prinzipiell zunächst eine Suspension des Materials für das schwammartige Gebilde hergestellt und anschließend gefriergetrocknet. Gegebenenfalls wird zuvor das Material für das schwammartige Gebilde zerkleinert und/oder einer alkalischen und/oder einer sauren Vorbehandlung unterworfen. Die Gefriertrocknung wird vorzugsweise bei -50 bis -150° C, insbesondere bei -80 bis -120° C durchgeführt.

Vor, während oder nach der Herstellung des schwammartigen Gebildes kann das Material mit den oben erwähnten pharmazeutisch wirksamen Stoffen, Nahrungsmitteln bzw. Nahrungsergänzungsmitteln Mineralstoffe, z.B. Vitaminen, Ballaststoffen, Eiweißen sowie andere Lebensmittelstoffen oder Aromastoffen beaufschlagt werden. Hierfür kommen alle üblichen Methoden in Betracht. Im einfachsten Falle kann dies während der Herstellungsphase des Schwammaterials durch Mischen von Trägermaterial und Wirkstoff erfolgen. Ebenso können Arzneistoffe, Aromastoffe oder Genußstoffe wie Schokolade, Zuckerguß oder ähnliche Stoffe auf der Oberfläche aufgebracht werden.

Zweck des erfindungsgemäßen Verfahrens ist es, ein Material zu erhalten, daß bei Durchtritt durch die Speiseröhre ausreichend komprimierbar ist und außerdem bei längerer Verweildauer in Magen nicht zersetzt wird. Dieses Ziel wird mit den genannten Verfahrensschritten erreicht. Im Gegensatz zu anderen Lebensmittel-/Nahrungsergänzungs-/Diät oder Arzneimittelprodukten, die kurzfristig vom Magensaft zersetzt werden oder schon in zerkleinertem Zustand in den Magen gelangen und somit in kurzer Zeit wieder den Magen passieren (verlassen), behält der aus natürlichen oder synthetischen Fasern bestehende, in der beschriebenen Weise hergestellte Schwamm- oder Schaumkörper durch besondere Faservernetzungsstellen über mehrere Stunden seine ursprünglich vorhandene Form, so daß der Magen erst nach mehreren Stunden passiert wird. Der Faserschwamm wird im Magen nicht resorbiert sondern unresorbiert ausgeschieden.

In einer Variante des erfindungsgemäßen Verfahrens kann lösliches Collagen aus den Häuten junger Rinder oder Schweine (Tieren) eingesetzt werden. Die löslichen Collagenanteile in der Haut von Tieren werden nämlich mit zunehmendem Alter des Organismus immer geringer, da das Collagen durch intermolekuläre Vernetzung ein unlösliches dreidimensionales Netzwerk bildet. Die Vemetzungsstellen sind feste chemische Bindungen zwischen einzelnen Collagenmolekülen.

Bei der Herstellung der notwendigen Collagensuspensionen für die Schwammherstellung müssen deshalb die Häute aus 1 bis 2 Jahre alten Tieren (Bullen) stammen. Auch hier bildet das Collagen schon ein unlösliches Netzwerk. Durch eine stark alkalische und saure Vorbehandlung der Haut und mechanische Kräfte beim Herstellen der Schwamm-Suspension, kann es dazu kommen, daß einzelne chemische und physikalische Vernetzungsstellen im Collagen gelöst werden.

Bei der Trockung des Schwamms durch Gefriertrocknung, vorzugsweise bei bis zu -120 °C, werden wieder neue Vernetzungsstellen durch die relativ hohen Temperaturen in das Schwammaterial eingeführt. Dies bewirkt die langandauernde Unlöslichkeit des Schwammkörpers im Magensaft. Diese relative Magensaftunlöslichkeit ist Voraussetzung für den durch den langen Aufenthalt des Schwamms im Magen anhaltenden Sättigungseffekt.

Die Erfindung ist nicht auf das beschriebene Verfahren beschränkt, sondern gilt auch für alle anderen Verfahren, bei denen Schwämme oder schwammähnlichen Gebilde hergestellt werden, die durch die relative Wasser- und Magensaftuntöslichkeit und die sich dadurch ergebende lange Verweildauer im Magen einen langfristigen Sättigungseffekt erzielen sollen oder können.

Das erfingungsgemäße Mittel wird oral eingenommen. Der feste Schwammoder feste Schaumkörper passiert durch Hinzufügen von Trinkflüssigkeit sowie leichte Kau- oder Schluckbewegungen die Mund- Rachen- und Speiseröhrenpassage und schwellt durch die Magenfiüssigkeit vorzugsweise zu seinem ursprünglichen Volumen im Magen wieder auf. Gegebenenfalls kann das Volumen auch größer oder kleiner als das ursprüngliche sein.

Durch die orale Einnahme des erfingungsgemäßen Mittels wird erreicht, daß der feste Schwamm- oder festen Schaumkörper durch die Schwerlöslichkeit im Magen über mehrere Stunden im Magen verweilt. Infolgedessen läßt sich ein langfristiges Sättigungs- oder Völlegefühl erzielen, das eine reduzierte Nahrungsaufnahme zur Folge hat.

Je nach gewünschtem Sättigungsgrad, können ein bis fünfzehn Schwammkörper in unterschiedlichen Zeitabständen täglich eingenommen werden. Die durch das im Magen befindliche Schwammvolumen angesprochenen "Sättigungssensoren" erzeugen über das Zwischenhirn einen Sättigungseffekt, der erst bei Leerung des Magens wieder zurückgeht. Somit kann durch die Länge des Aufenthalts und die Größe der Volumina der Schwämme die Sättigungsdauer gesteuert werden

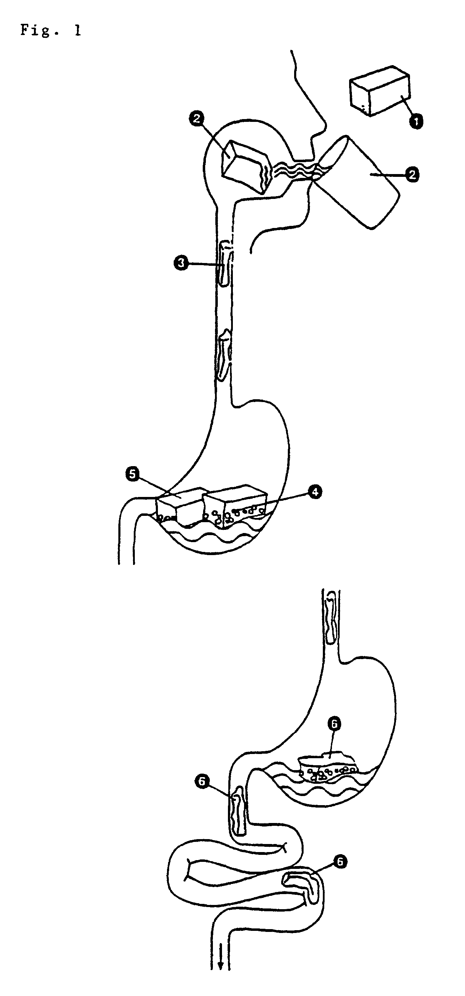

Im folgenden wird die Erfindung unter Bezugnahme auf die Figur näher erläutert:
1. Magensaft- und wasserresistenter Schaumwürfel
2. Durch Zuführung von etwas Flüssigkeit verliert der trockene Schaumwürfel seine feste Form und läßt sich in seiner wäßrig-glitschigen Form wie andere Lebensmittel problemlos schlucken.
3. Der glitschig deformierte Schaumwürfel passiert die Speiseröhre.
4. Im Magen angelangt, saugt der Schwamm die dort befindliche Magen- und Trinkflüssigkeit auf und nimmt sein altes Raumvolumen wieder ein
5. Der durch seine besondere Faserstruktur nicht verdaubare Schaumwürfel verweilt durch Beibehaltung seines Volumens 2-20 Stunden im Magen. Durch sein Volumen wird der Magenausgang nicht passiert.
6. Erst durch die ständige Magenbewegung (Peristaltik, 1-20 Stunden)und das Einwirken der Magensäure verliert der Schwamm wieder seine Würfelform (ohne resorbiert zu werden) und hat dadurch erst nach 1-20 Stunden die Möglichkeit, den Magenausgang zu passieren, um über den Darm abgeführt zu werden. Normale Lebensmittel, wie Fleisch oder Gemüse, werden bereits nach kurzer Zeit von der Magensäure zerkleinert und aufgelöst, um dann auch nach wenigen Minuten den Magenausgang zu passieren. Durch die beschriebenen besondere Faserstruktur behält hingegen das Mittel gemäß der Erfindung durch die Magensaftresistenz im Magen ihr Ursprungsvolumen, so daß der stundenlange und somit sättigende Magenaufenthalt biologisch (physiologisch) bedingt ist

## Patentansprüche

1. Mittel zur oralen Einnahme, enthaltend ein in Wasser und gastrointestinalen Flüssigkeiten unlösliches oder schwerlösliches Material in Form eines schwammartigen Gebildes,
**dadurch gekennzeichnet, daß** es aus einem elastischen Material besteht,
- das durch Kau- und Gaumenschluckbewegung verformbar, formreduzierbar und Komprimierbar, so daß es die Speiseröhre passieren kann und
- das nach dem Verlassen der Speiseröhre in Wasser und gastrointestinalen Flüssigkeiten im Magen Volumen aufbauend dekomprimierbar ist.

2. Mittel nach Anspruch 1,
**dadurch gekennzeichnet, daß** es nach dem Verlassen der Speiseröhre auf annähernd die Größe dekomprimierbar ist, die es vor eintritt in die Speiseröhre durch orale Einnahme aufgewiesen hat.

3. Mittel nach einem der Ansprüche 1 oder 2,
**dadurch gekennzeichnet, daß** das schwammartige Gebilde auf1/2 bis 1/20 vorzugsweise 1/4 bis 1/10 seiner Größe komprimierbar ist

4. Mittel nach einem der Ansprüche 1 bis 3,
**dadurch gekennzeichnet, daß** das schwammartige Gebilde nach dem Verlassen der Speiseröhre auf das Zwei - bis Zwanzigfache, vorzugsweise das Vier - bis Zehnfache seiner Größe im komprimierten Zustand dekomprimierbar ist.

5. Mittel nach einem der Ansprüche 1 bis 4,
**dadurch gekennzeichnet, daß** das Material für das schwammartige Gebilde Viskoseschwämme sind.

6. Mittel nach einem der Ansprüche 1 bis 5,
**dadurch gekennzeichnet, daß** das Material für das schwammartige Gebilde aus natürlichen, halbsynthetischen oder synthetischen Polymeren besteht.

7. Mittel nach einem der Ansprüche 1 bis 6,
**dadurch gekennzeichnet, daß** das Material für das schwammartige Gebilde aus langfaserigen, linear kolloiden, hochmolekularen Polymeren besteht.

8. Mittel nach Anspruch 7,
**dadurch gekennzeichnet, daß** das Material für das schwammartige Gebilde aus Polymeren mit Faserstruktur, vorzugsweise aus Proteinen besteht.

9. Mittel nach Anspruch 8,
**dadurch gekennzeichnet, daß** das Material für das schwammartige Gebilde aus Skleroproteinen, vorzugsweise Collagenen besteht.

10. Mittel nach einem der Ansprüche 1 bis 9,
**dadurch gekennzeichnet, daß** das schwammartige Gebilde Nahrungs - und/oder Arzneimittel b.z.w. Wirkstoffe oder Aromastoffe enthält und/oder äußerlich mit Arzneistoffen, Lacken oder Lebensmittelstoffen (Genußstoffen) wie Schokolade, Zuckerguß, Fruchtmasse, u.s.w. beschichtet oder überzogen ist

11. Verfahren zur Herstellung des Mittels nach einem der Ansprüche 1 bis 10,
**dadurch gekennzeichnet, daß** eine collagenhaltige Suspension des Materials für das schwammartige Gebilde hergestellt und anschließend gefriergetrocknet wird.

12. Verfahren zur Herstellung des Mittels nach einem der Ansprüche 1 bis 11,
**dadurch gekennzeichnet, daß** das Material für das schwammartige Gebilde zerkleinert und/oder einer alkalischen und/oder einer sauren Vorbehandlung unterworfen wird.

13. Verfahren zur Herstellung des Mittels nach einem der Ansprüche 1 bis 12,
**dadurch gekennzeichnet, daß** die Erhitzung bei der Gefriertrocknung bei -50° bis -150° C, vorzugsweise bei -80 bis -120° C durchgeführt wird.

14. Verwendung des Mittels nach einem der Ansprüche 1 bis 13 zur Herstellung von Mitteln zur Erzeugung eines Sättigungseffekts sowie zur Herstellung von oral verabreichbaren Arzneimitteln, Nahrungsergänzungsmittlen, Nahrungsmitteln oder Genußmitteln mit zeitverzögerter und schonender Wirkstoff- und Inhaltfreisetzung.

## Claims

1. Composition for oral ingestion, comprising a material which is insoluble or poorly soluble in water and gastrointestinal fluids, in the form of a sponge-like structure, **characterized in that** it consists of an elastic material,
- which is deformable, shape-reducible and compressible by chewing and palatine swallowing motion so that it can pass through the oesophagus and
- which, after leaving the oesophagus, is decompressible in water and in gastrointestinal fluids in the stomach in a volume-building manner.

2. Composition according to Claim 1, **characterized in that** after leaving the oesophagus it is decompressible to approximately the size which it had before entry into the oesophagus by oral ingestion.

3. Composition according to one of Claims 1 or 2, **characterized in that** the sponge-like structure is compressible to 1/2 to 1/20th, preferably 1/4 to 1/10th, its size.

4. Composition according to one of Claims 1 to 3, **characterized in that** the sponge-like structure is decompressible after leaving the oesophagus to two- to twenty-fold, preferably four- to ten-fold, its size in the compressed state.

5. Composition according to one of Claims 1 to 4, **characterized in that** the materials for the sponge-like structures are viscose sponges.

6. Composition according to one of Claims 1 to 5, **characterized in that** the material for the sponge-like structure consists of natural, semi-synthetic or synthetic polymers.

7. Composition according to one of Claims 1 to 6, **characterized in that** the material for the sponge-like structure consists of long-fibre, linear colloidal, high molecular weight polymers.

8. Composition according to Claim 7, **characterized in that** the material for the sponge-like structure consists of polymers with fibre structure, preferably of proteins.

9. Composition according to Claim 8, **characterized in that** the material for the sponge-like structure consists of scleroproteins, preferably collagens.

10. Composition according to one of Claims 1 to 9, **characterized in that** the sponge-like structure contains foodstuffs and/or medicaments or active compounds or flavourings and/or is coated or covered externally with pharmaceuticals, lacquers or foodstuffs (luxury foods) such as chocolate, icing sugar, fruit material, etc.

11. Process for the preparation of the composition according to one of Claims 1 to 10, **characterized in that** a collagen-containing suspension of the material for the sponge-like structure is prepared and then freeze-dried.

12. Process for the preparation of the composition according to one of Claims 1 to 11, **characterized in that** the material for the sponge-like structure is comminuted and/or subjected to an alkaline and/or an acidic pretreatment.

13. Process for the preparation of the composition according to one of Claims 1 to 12, **characterized in that** the heating during the freeze-drying is carried out at -50°C to -150°C, preferably at -80 to -120°C.

14. Use of the composition according to one of Claims 1 to 13 for the preparation of compositions for producing a satiation effect and for the preparation of orally administrable medicaments, food supplements, foodstuffs or luxury foods having a time-delayed and gentle release of active compound and contents.

## Revendications

1. Produit destiné à la prise orale, contenant un matériau sous forme d'une structure spongieuse insoluble ou difficilement soluble dans l'eau et les liquides gastro-intestinaux, **caractérisé en ce qu'**il est constitué d'un matériau élastique
- qui est déformable, réductible quant à la forme et comprimable sous l'effet de mouvement de déglutition et de mastication, de sorte qu'il peut passer dans l'oesophage, et
- qui, après avoir quitté l'oesophage, peut dans l'estomac se décomprimer dans l'eau et les liquides gastro-intestinaux en augmentant de volume.

2. Produit selon la revendication 1, **caractérisé en ce que**, après avoir quitté l'oesophage, il peut se décomprimer jusqu'à approximativement la taille qu'il a avant l'entrée dans l'oesophage par prise orale.

3. Produit selon la revendication 1 ou 2,
**caractérisé en ce que** la structure spongieuse est comprimable jusqu'à ½ à 1/20, de préférence ¼ à 1/10 de sa taille.

4. Produit selon la revendication 1 ou 3,
**caractérisé en ce que**, après avoir quitté l'oesophage, la structure spongieuse peut se décomprimer jusqu'à deux à vingt fois, de préférence quatre à dix fois sa taille à l'état comprimé.

5. Produit selon l'une quelconque des revendications 1 à 4, **caractérisé en ce que** le matériau pour la structure spongieuse est constitué d'éponges de viscose.

6. Produit selon l'une quelconque des revendications 1 à 5, **caractérisé en ce que** le matériau pour la structure spongieuse est constitué de polymères naturels, semi-synthétiques ou synthétiques.

7. Produit selon l'une quelconque des revendications 1 à 6, **caractérisé en ce que** le matériau pour la structure spongieuse est constitué de polymères de masse moléculaire élevée, colloïdaux linéaires à longues fibres.

8. Produit selon la revendication 7, **caractérisé en ce que** le matériau pour la structure spongieuse est constitué de polymères à structure fibreuse, de préférence de protéines.

9. Produit selon la revendication 8, **caractérisé en ce que** le matériau pour la structure spongieuse est constitué de scléroprotéines, de préférence de collagène.

10. Produit selon l'une quelconque des revendications 1 à 9, **caractérisé en ce que** la structure spongieuse contient des aliments et/ou des médicaments ou des substances actives ou des arômes et/ou est enduite ou revêtue avec des médicaments, des vernis ou des produits alimentaires (friandises) tels que le chocolat, un produit couvert de sucre glace, un produit à base de fruits, etc.

11. Procédé pour la fabrication du produit selon l'une quelconque des revendications 1 à 10, **caractérisé en ce qu'**une suspension, contenant du collagène, du matériau pour la structure spongieuse est préparée et ensuite lyophilisée.

12. Procédé pour la fabrication du produit selon l'une quelconque des revendications 1 à 11, **caractérisé en ce que** le produit pour la structure spongieuse est fragmenté et/ou soumis à un prétraitement alcalin et/ou un prétraitement acide.

13. Procédé pour la fabrication du produit selon l'une quelconque des revendications 1 à 12, **caractérisé en ce que** la lyophilisation est effectuée à une température de -50° à -150°C, de préférence de -80 à -120°C.

14. Utilisation du produit selon l'une quelconque des revendications 1 à 13, pour la fabrication de produits destinés à procurer un effet de satiété, ainsi que pour la fabrication de médicaments, compléments nutritionnels, produits alimentaires ou friandises pouvant être absorbés par voie orale, à libération ralentie et ménagée du contenu et de la substance active.
